# EUROPEAN PATENT SPECIFICATION

(11) **EP 3 172 311 B1**
(45) Date of publication and mention of the grant of the patent: **02.10.2019**
(21) Application number: 15741224.8
(22) Date of filing: 24.07.2015
(51) Int. Cl.: C12M 1/04, C12M 1/06, C12M 1/26, C12N 1/06

(54) **METHOD AND APPARATUS FOR DISRUPTION OF BIOMASS CELLS**
VERFAHREN UND VORRICHTUNG ZUR SPALTUNG VON BIOMASSEZELLEN
PROCÉDÉ ET APPAREIL POUR LA DÉSAGRÉGATION DE CELLULES DE BIOMASSE

(30) Priority: 25.07.2014 EP 14178628
(43) Date of publication of application: 31.05.2017
(73) Proprietor: VITO NV (Vlaamse Instelling voor Technologisch Onderzoek NV), 2400 Mol (BE)
(72) Inventor: D'HONDT, Els, 2400 Mol (BE); ELST, Kathy, 2400 Mol (BE); GÜNERKEN, Emre, 2400 Mol (BE); GARCIA-GONZALEZ, Linsey, 2400 Mol (BE); WIJFFELS, René H., 6700 AA Wageningen (NL); EPPINK, Michel H.M., 6700 AA Wageningen (NL)
(74) Representative: Pronovem
(86) International application number: PCT/EP2015/067056
(87) International publication number: WO 2016/012613

(56) References cited:
- WO-A1-91/01367
- WO-A2-2004/108260
- US-A- 5 306 637
- US-A1- 2011 183 403

## Description

### Field of the Invention

The present invention relates to a sparger system which can be used in a method and apparatus for disrupting suspended biomass material.

The sparger system of the present invention can also be used in a method for solubilizing (valuable) cellular substances (from disrupted biomass material), and a related apparatus (and stack of (parallel) systems).

Using the sparger system of the present invention allows a subsequent selective extraction (or separation) of the obtained cellular substances (from the disrupted biomass material).

### Background of the Invention

To exploit a range of bioproducts from biological sources (including bacteria, yeast, algae, plant and animal tissues), liberation of intracellular products is of major importance where these products are not secreted into the suspending media by the microbial construct. The cell wall provides structural strength and is typically the key resistance to product liberation. The cell membrane, in turn, is readily disrupted in the absence of the cell wall.

In the art, a variety of disruption techniques are already available for the release of intracellular products.

Known mechanical cell disruption methods are, for example, ball milling, bead milling, high-pressure homogenizing, high-speed homogenizing, pressing of a sample at high pressure through a narrow aperture, or a method applied by means of an ultrasonic homogenizer.

A major drawback of these mechanical methods is the shear forces acting on the cells due to friction which, when trying to overcome them at high velocities, lead to a formation of heat and consequently generate a high temperature. In addition to high temperature, cavitational cell disruption methods (such as high speed homogenizing, high pressure homogenizing and ultrasonic homogenizer) cause extreme pressure differences. These may entail a harmful impact on the constituents of the evolving cellular extracts.

A more gentle method is for example provided by a physical decompression of cells, known in the art as (conventional) explosive decompression method. According to Henry's law, gas in cells is enriched at higher gas pressures and the cell membranes are thus caused to burst due to an abrupt pressure relief. This occurs because the dissolved gas cannot escape fast enough and bubbles out within the cells in form of growing gas bubbles. As a result, the mechanical load acting on the cell rises until the cell bursts and the cell content is released.

However, a drawback of the conventional explosive decompression method lies in that only cells that are relatively easy to break up can be efficiently disrupted which calls for additional application of non-mechanical disruption processes, like the use of enzymes. This in turn renders such a disruption process non-profitable for large-scale applications.

Mechanical methods have found greater commercial application compared with non-mechanical methods, because the latter infer operational and economic limitations at process scale. However, the disadvantages of the use of mechanical methods include a non-selective release of the product and micronization of the cell debris, complicating the further downstream process.

It is important to further integrate cell disruption with the formation and recovery (extraction) of the intracellular products.

Extraction of intracellular products is known in the art to be executed, for example, by means of supercritical fluids. This term relates to gases or liquids which are above their critical temperature and critical pressure that are defined in the relevant phase diagram of a pure substance. The benefit lies in an increased solubility for hardly soluble substances in the supercritical range. Moreover, solubility can still be controlled via alterations in pressure or temperature. However, the solubility of the supercritical form of a substance in water is generally lower than the solubility of the same substances in liquid form. Thus usually totally dried biomass is used in supercritical extraction methods known in the art.

An example is given by the decaffeination of a tea plant by means of supercritical CO₂, as described in WO 2008/05537 A1. Other applications in chemical industry and foodstuff industry have become well established methods as well, for example the extraction of oils, ginger, black pepper or chili powder by means of supercritical CO₂ or supercritical propane.

U.S. Patent No. 5,306,637 describes a method for cell disruption in which an enzymatic disruption is linked to a subsequent abrupt pressure relief causing the cells to burst and effectively releasing the valuable substance contained in them. U.S. Patent No. 5,306,637 uses supercritical or almost supercritical carbon dioxide, to which entraining agents are optionally added, for the disruption of microbial cells and for the extraction of the intracellular components, such as proteins or nucleic acids. However, here, the recovery of the used gas mixtures is extremely problematic and, therefore, this process is not applied on an industrial scale.

US 2011/0183403 describes a method for cell disruption of biogenic suspended raw materials by means of a combination of pressurization, atomization and decompression including a subsequent selective extraction and separation of cellular valuable substances. By this method, it is achieved that the cell disruption of the biogenic suspended raw material and the dissolution of the cellular valuable substances are performed simultaneously.

### Aims of the Invention

Aspects of the present invention envisage providing an improved sparger system which can be used in a method for disrupting suspended biomass material and a related apparatus (and stack of (parallel) systems), which overcome the disadvantages of prior art methods and apparatuses.

More particularly, it is envisaged to provide a sparger system which can be used in a method and a related apparatus (and stack of (parallel) systems) for a physical (non-mechanical), mild disruption of suspended biomass material.

### Summary of the Invention

According to aspects of the invention, there is therefore provided a sparger system, as set out in the appended claims.

Advantageous aspects of the present invention are set out in the dependent claims.

### Short Description of the Drawings

Aspects of the invention will be described in more detail with reference to the appended drawings, wherein same reference numerals illustrate same features. In the drawings, not all alternatives and options are shown and therefore the invention is not limited to the content of the given drawings.
**Figure 1a** schematically represents an apparatus for disrupting suspended biomass material and, optionally, solubilizing the (valuable) cellular substances, the apparatus comprising a sparger system (7) according to an aspect of the present invention.
**Figure 1b** schematically represents an apparatus for disrupting suspended biomass material and, optionally, solubilizing the (valuable) cellular substances, the apparatus comprising a sparger system (7) according to an aspect of the present invention, said apparatus further comprising a pressure oscillation system (9).
**Figure 2** schematically represents a sparger system (7) of the present invention, used in the apparatus of **figures 1a** and **1b****.**
**Figure 3** depicts the comparison of the solubilization ratios (%) of different biochemical compositions, for untreated and treated suspensions of biomass using methods known in the art and a method according to an aspect not according to the present invention but useful for the understanding thereof.
**Figure 4** represents the energy consumption (in kWh/kg of dry biomass) versus the CO₂/suspension of biomass material ratio using the disruption method according to an aspect not according to the invention but useful for the understanding thereof.

### Description of the Invention

According to an aspect not according to the invention but useful for the understanding thereof, there is provided an apparatus for disrupting suspended biomass material.

In the context of the present invention, disruption of (suspended, treated) biomass material refers to disruption of biomass cells. In the present description, this is also referred to as cell disruption. More particularly, during said disruption cell membranes as well as intracellular membranous structures are being disrupted. Intracellular membrane structures are, for example, thylakoid membranes or endoplasmic reticulum membranes.

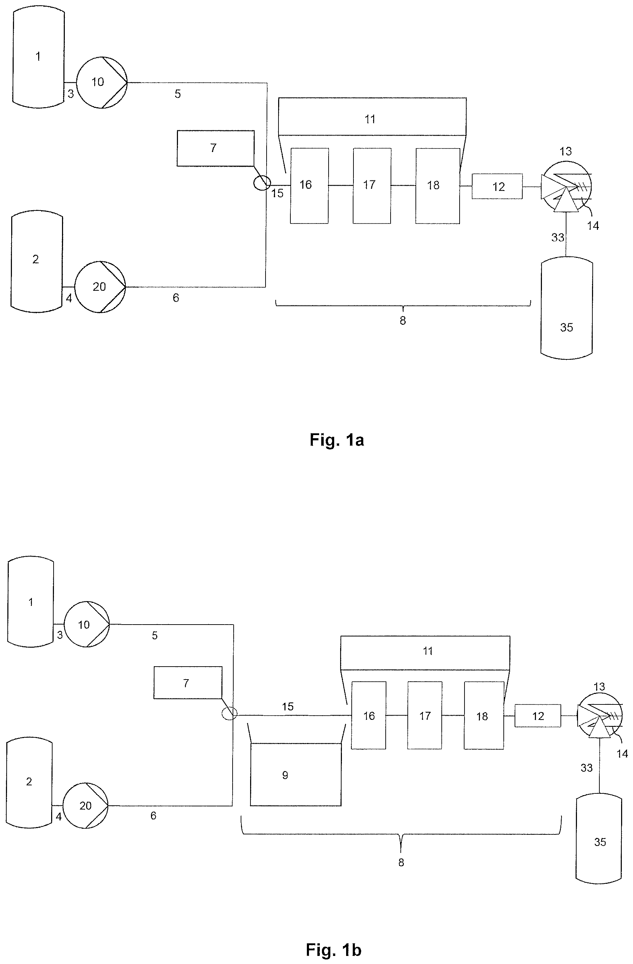

As illustrated schematically in **figure 1a****,** an apparatus using a sparger system according to the present invention comprises (or consists of) at least one first vessel (1) for holding (or providing) a (untreated) suspension of biomass material connected to at least one first high-pressure pump (10) by at least one tube (3), said first high-pressure pump (10) being adapted for pressurizing the suspension of biomass material coming from (supplied by) said first vessel (1); at least one second vessel (2) for holding (or providing) a (pressurized) gas disruption agent and/or (pressurized) liquid disruption agent connected to at least one second high-pressure pump (20) by at least one tube (4), said second high-pressure pump (20) being adapted for (further) pressurizing the gas and/or liquid disruption agent coming from (supplied by) said second vessel (2); wherein each of said first and second high-pressure pump (10,20) is connected by at least one tube (5,6) to at least one sparger system (7) according to the present invention, said sparger system (7) being adapted for injecting a first stream (provided through said at least one tube (6)) of pressurized disruption agent into a second stream (provided through said at least one tube (5)) of a pressurized suspension of biomass material and for forming a micro-emulsion, wherein said sparger system (7) is further connected to a tubing system (8) for mixing said first and second streams, said tubing system (8) comprising a (at least one) primary tube (15) and (a) at least one hydrodynamic agitation system (11), and said tubing system (8) being further connected to a relief valve (13).

In the present description, a disruption (or disrupting) agent refers to a chemical compound used for disrupting biomass cells.

The gas and/or liquid disruption agent coming from (supplied by) said second vessel (2) is only present in liquid phase (or liquid state) after having passed second high-pressure pump (20), i.e. the pressure applied by second high-pressure pump (20) is such that no gas disruption agent is present in the system any more after having passed second high-pressure pump (20)). Said first stream of pressurized disruption agent is thus only present in liquid phase (or liquid state, i.e. being liquefied).

The disruption agents provided in second vessel (2) and the temperatures and pressures applied are such that no supercritical fluids can be present in the system until and/or after the relief valve (13). Optionally, the temperature of the obtained micro- and/or nano-emulsion is only increased to the subcritical or critical point of the disruption agent, while transferring the process stream coming out of the relief valve (13) to a third vessel (35) at reduced or atmospheric pressure for (subsequently) solubilizing the (valuable) cellular substances (or cellular extracts) (from the disrupted biomass material).

Advantageously, the reduced pressure is comprised between (about) atmospheric pressure and the pressure (in the system) in between the hydrodynamic agitation system (11) and relief valve (13).

Advantageously, in an apparatus comprising a sparger system (7) according to the present invention, said sparger system (7) is being adapted for injecting a first stream (provided through said at least one tube (6)) of pressurized disruption agent into a second stream (provided through said at least one tube (5)) of a pressurized suspension of biomass material and for forming a micro- and/or nano-emulsion.

In other words, an apparatus comprising a sparger system (7) according to the invention as illustrated schematically in **figure 1a****,** comprises at least two high-pressure pumps (10,20), wherein at least one first pump (10) serves as high-pressure slurry pump and at least one second pump (20) serves as high pressure liquid/gas pump. Said pumps (10,20) each contain at least one inlet and at least one outlet. Said first pump (10) and second pump (20) are being configured (adapted) to pressurize a suspension of biomass material (or slurry) and a gas/liquid disruption agent, respectively, and to feed them further to the system (7) via tubes (5,6). Furthermore, the apparatus comprises at least one first vessel (1), serving as the feed source for an untreated suspension of biomass material, said at least one first vessel (1) having an outlet connected to the inlet of the high pressure slurry pump (10) via a tube (or conduit) (3). The apparatus comprises also at least one second vessel (2), serving as the feed source for pressurized gas/liquid disruption agent, said at least one second vessel (2) having an outlet connected to the inlet of high pressure liquid/gas pump (20) via a tube (or conduit) (4). The apparatus further comprises at least one sparger system (7) according to the present invention serving as the injector of pressurized (liquefied) disruption agent into a pressurized suspension of untreated biomass material (or adapted (configured) for injecting a pressurized (liquefied) disruption agent stream coming from (supplied by) high pressure liquid/gas pump (20) into the pressurized suspension of biomass material stream coming from (supplied by) high pressure slurry pump (10)) and being adapted for forming a micro- and/or nano-emulsion. The sparger system (7) is then sequentially connected by a primary tube (15) to at least one hydrodynamic agitation system (11) and a relief valve (13).

In the context of the present invention, the wording gas/liquid disruption agent refers to gas disruption agent (or disruption agent being in gas phase) and/or liquid disruption agent (or disruption agent being in liquid phase). In other words, depending on the temperature and/or the pressure in the system, the disruption agent in second vessel (2) is present in gas phase (or gas state) and/or in liquid phase (or liquid state).

In the context of the present invention, a stream of pressurized disruption agent coming out of the second high-pressure pump (20) (flowing through tube (6) to sparger system (7)) is only present in liquid phase (or liquid state, being liquefied) (i.e. the pressure applied by second high-pressure pump (20) is such that no gas disruption agent is present in the system any more after having passed second high-pressure pump (20)).

Advantageously, said first vessel (1) is connected to said first high-pressure pump (10) by a tube (3).

Advantageously, said second vessel (2) is connected to said second high-pressure pump (20) by a tube (4).

Advantageously, the at least one second vessel (2) is a tight-sealable vessel (i.e. being sealed off with respect to the surroundings).

According to an aspect not according to the present invention (but useful for the understanding thereof), an apparatus schematically illustrated in **figure 1a** can be used for disrupting suspended biomass material.

Furthermore, according to an aspect of the invention, there is provided a sparger system.

A sparger system of the invention is being adapted for injecting a first stream of pressurized (liquefied) disruption agent into a second stream of a pressurized suspension of biomass material so as to form a (mixed stream of) micro- and/or nano-emulsion (of disruption agent with biomass suspension).

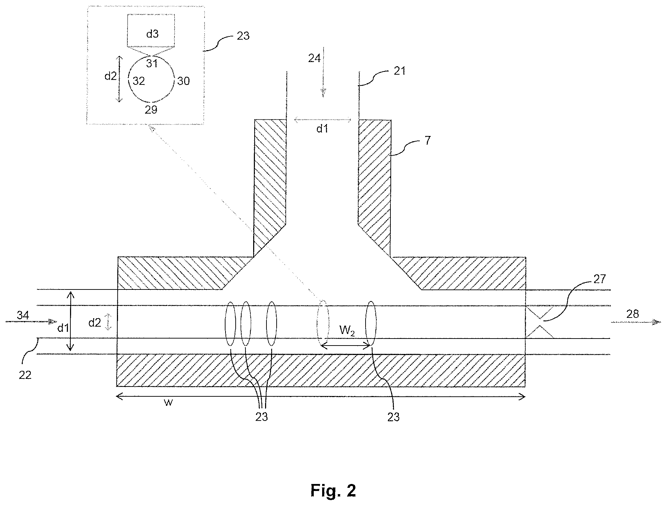

A sparger system (7) according to the present invention is schematically illustrated in **figure 2****.**

The sparger system of the invention comprises a first tube (21) comprising a first inlet (24) for supplying a stream of pressurized disruption agent into the tube (21); a second tube (22) comprising a second inlet (34) for supplying a stream of a pressurized suspension of biomass material through the tube (22), the second tube (22) comprising an outlet (28), wherein the second tube (22) traverses the first tube (21) and comprises at least one set (23) of first openings (29-32) through a wall of the tube (22), the first openings (29-32) being in fluid communication with the first tube (21) such that a micro-emulsion is formed by forcing the stream of pressurized disruption agent through the first openings (29-32) into the second tube (22), wherein the first openings (29-32) have a diameter (d3) between 0.01 mm and 1 mm.

Advantageously, the first tube (21) is dead ending on the second tube (22).

Advantageously, the second tube (22) has a (second) diameter (d2) being two times smaller than the (first) diameter (d1) of the first tube (21), i.e. the ratio of the diameter (d2) of the second tube (22) to the diameter (d1) of the first tube (21) is 0.5.

Preferably, the sparger system (7) comprises a first sparger tube (21) having a (first) diameter (d1) and having two inlets (24,34) and one outlet (28). Said first sparger tube (21) comprises a second sparger tube (22) placed along the direction formed by one of the inlets (34) and the outlet (28) (i.e. the second tube (22) traverses the first tube (21)), said second sparger tube (22) having a (second) diameter (d2) being two times smaller than the (first) diameter (d1) of the first sparger tube (21) (i.e. the ratio (d2)/(d1) is 0.5). Said second sparger tube (22) comprises a distribution of sets (23) of first openings through the surface (wall) of the second sparger tube (22) (the sets (23) of first openings in fluid communication with the first tube (21)) along a width (w) (of first sparger tube (21)), said first openings having a (third) diameter (d3) comprised between (about) 0.01 mm and (about) 1 mm, more preferably between (about) 0.01 mm and (about) 0.2 mm, even more preferably between (about) 0.025 mm and (about) 0.085 mm, most preferably between (about) 0.05 mm and (about) 0.06 mm.

In the present description, said first sparger tube (21) can also be referred to as sparger shell (21).

Preferably, said first sparger tube (21) in sparger system (7) of the invention is T-shaped or Y-shaped, more preferably said first sparger tube (21) is T-shaped.

Advantageously, said second sparger tube (22) comprises one, two, three, or more sets (23) of first openings through the surface (wall) of the second sparger tube (22) along a width (w) (of sparger shell (21)).

More advantageously, the value of width (w) is comprised between the value of (third) diameter (d3) of the first openings through the surface (wall) of the second sparger tube (22) and the total length of the second sparger tube (22) being inside (comprised within) first sparger tube (21). Even more advantageously, the value of width (w) is as small as possible to comprise all sets (23) of first openings (next to each other, along the width (w)), i.e. the value of width (w) satisfying the equation w=Σw₂.

More advantageously, said second sparger tube (22) comprises (only) one set (23) of first openings through the surface (wall) of the second sparger tube (22). In other words, (only) one set (23) of first openings is provided through the surface (wall) of the second sparger tube (22).

Without being bound by theory, providing more than two first openings (i.e. four, six, or more) per set of first openings, and/or providing more than one set of first openings (i.e. two, three, or more), will change the superficial velocity proportional to the parameter (1/the number of first openings) and the ratio of (first opening diameter (d3))²/(second sparger tube diameter (d2)²). Preferably, the parameter (1/the number of openings) is chosen smaller than the ratio of (first opening diameter (d3))²/(second sparger tube diameter (d2)²). In this preferable case, the superficial velocity of disruption agent will increase, which will lead to a pressure drop in the (second sparger tube (22) of) the sparger system (7). If the pressure drop due to the disruption agent passing through said first openings is higher than 50% of the pump pressure of the disruption agent (said pump pressure being provided by the second high-pressure pump (20) pressurizing the disruption agent coming from (supplied by) second vessel (2) (cf. e.g. **figure 1a**)), then, more advantageously, said second sparger tube (22) comprises more than one set (23) of first openings through the surface (wall) of the second sparger tube (22) (i.e. then, more advantageously, more than one set (23) of first openings is provided through the surface (wall) of the second sparger tube (22)).

Even more advantageously the value of width (w) equals (or is very close to) the value of (third) diameter (d3) of the first openings through the surface (wall) of the second sparger tube (22).

Preferably, said second sparger tube (22) further comprises a second opening (27) at the outlet (28) of the second sparger tube (22), said second opening (27) having a diameter (d4) comprised between (about) 0.01 mm and (about) 0.6 mm, more preferably between (about) 0.1 mm and (about) 0.6 mm.

Advantageously, said first high-pressure pump (10) is connected by a tube (5) to the sparger system (7) (cf. e.g. **figure 1a**). More particularly, tube (5) is connected to inlet (34) of the sparger system (7). More particularly, first high-pressure pump (10) is connected to inlet (34) of the sparger system (7). More particularly, second tube (22) comprises a second inlet (34) for supplying a stream of a suspension of biomass material through the tube (22).

Advantageously, said second high-pressure pump (20) is connected by a tube (6) to the sparger system (7). More particularly, tube (6) is connected to inlet (24) of the sparger system (7). More particularly, second high-pressure pump (20) is connected to inlet (24) of the sparger system (7). More particularly, first tube (21) comprises a first inlet (24) for supplying a stream of disruption agent into tube (21).

By using the sparger system (7) of the invention, a first stream (provided through said at least one tube (6)) of pressurized disruption agent is injected into a second stream (provided through said at least one tube (5)) of a pressurized suspension of biomass material and a micro- and/or nano-emulsion is formed.

Preferably, (first) diameter (d1) of first sparger tube (21) is comprised between (about) 1.6 mm (1/16 inch) and (about) 25.4 mm (1 inch), more preferably between (about) 3.2 mm (1/8 inch) and (about) 12.7 mm (1/2 inch), even more preferably (first) diameter (d1) of first sparger tube (21) is (about) 6,4 mm (1/4 inch).

Preferably, (second) diameter (d2) of second sparger tube (22) is comprised between (about) 0.8 mm (1/32 inch) and (about) 12.7 mm (1/2 inch), more preferably between (about) 1.6 mm (1/16 inch) and (about) 3.2 mm (1/8 inch), even more preferably (second) diameter (d2) of second sparger tube (22) is (about) 3.2 mm (1/8 inch).

Preferably, diameter (d5) of tube (5) is comprised between (about) 0.8 mm (1/32 inch) and (about) 12.7 mm (1/2 inch), more preferably between (about) 1.6 mm (1/16 inch) and (about) 3.2 mm (1/8 inch).

Preferably, diameter (d6) of tube (6) is comprised between (about) 0.8 mm (1/32 inch) and (about) 12.7 mm (1/2 inch), more preferably between (about) 1.6 mm (1/16 inch) and (about) 3.2 mm (1/8 inch).

Advantageously, each set (23) of first openings in second sparger tube (22) comprises an even number (i.e. 2, 4, 6, or more) of first openings (or first orifices), said openings being located two by two opposite each other in one plane, said plane being perpendicular to the second sparger tube (22), each of the first openings being located through the surface (wall) of the second sparger tube (22).

More advantageously, each set (23) of first openings in second sparger tube (22) comprises at least two first openings (or first orifices) (29,31), said openings being located opposite each other in one plane, said plane being perpendicular to the second sparger tube (22), the first openings (29,31) being located through the surface (wall) of the second sparger tube (22) at a distance D1 satisfying the equation D1=(diameter (d2) of second sparger tube x π)/2 from the next opening of the set (or first openings (29,31) placed 180° from each other). Even more advantageously, each set (23) of first openings in second sparger tube (22) consists of two first openings (or first orifices) (29,31).

Even more advantageously, each set (23) of first openings in second sparger tube (22) comprises (or consists of) (only) two first openings (or first orifices) (29,31). In other words, (only) two first openings (29,31) are provided in each set (23) of first openings in second sparger tube (22).

More advantageously, each set (23) of first openings in second sparger tube (22) comprises at least four first openings (or first orifices) (29,30,31,32), said openings being located two by two opposite each other in one plane, said plane being perpendicular to the second sparger tube (22), each of the first openings (29,30,31,32) being located through the surface (wall) of the second sparger tube (22) at a distance D1 satisfying the equation D1=(diameter (d2) of second sparger tube x π)/4 from the next opening of the set (orfirst openings (29,30,31,32) placed 90° from each other). Even more advantageously, each set (23) of first openings in second sparger tube (22) consists of four first openings (or first orifices) (29,30,31,32).

Even more advantageously, each set (23) of first openings in second sparger tube (22) comprises (or consists of) (only) four first openings (or first orifices) (29,31). In other words, (only) four first openings (29,31) are provided in each set (23) of first openings in second sparger tube (22).

Advantageously, the first openings (29,30,31,32) are made by laser drilling.

In the present description, first openings (29,30,31,32) in second sparger tube (22) are also referred to as first orifices, (first) sparger holes, or (first) sparging holes.

In the present description, (third) diameter (d3) of the first openings (29,30,31,32) in second sparger tube (22) is also referred to as the sparging hole diameter (of the sparging holes).

Advantageously, the sparger system (7) of the invention is a high-pressure sparger system.

In the present invention, the sparger system (7) serves as the injector of pressurized (liquefied) disruption agent into a pressurized suspension of biomass material. In other words, the sparger system (7) is adapted (configured) for injecting a first stream of pressurized (liquefied) disruption agent coming from (supplied by) high pressure liquid/gas pump (20) into a second stream of pressurized suspension of biomass material coming from (supplied by) high pressure slurry pump (10), thereby bringing (mixing) said first and second streams together (said streams being at high pressure) forming one (mixed) process stream.

In the context of the present invention, mixing refers to bringing two streams together, thereby bringing the two streams (only) in physical contact with each other. In other words, mixing is performed for (physically) "bringing together" two streams, without (chemical) reaction between said two streams, thereby forming one (mixed) process stream.

Advantageously, the first openings (29,30,31,32) in said second sparger tube (22) are adapted (configured) for producing micro (liquefied) disruption agent droplets (bubbles) during said injection, thereby producing (forming) a micro-emulsion of mixed (liquefied) disruption agent with biomass suspension inside the sparger. Accordingly, using the sparger of the present invention, there is a relatively high efficiency of mass transfer between the (liquefied) disruption agent and the suspension to be mixed in the sparger. In the micro-emulsion, micro droplets (bubbles) of (liquefied) disruption agent (the dispersed phase) are dispersed in the suspension of biomass material (the continuous phase or dispersion medium).

In the present description, the stream of pressurized disruption agent coming out of the second high-pressure pump (20) (flowing further to the sparger system (6)) is referred to as 'liquefied disruption agent'. In other words, the pressure applied by second high-pressure pump (20) is such that no gas disruption agent is present in the system any more after the disruption agent having passed second high-pressure pump (20) (disruption agent is thus only present in liquid phase (or liquid state) after having passed second high-pressure pump (20)).

Advantageously, the sparger system (7) according to the invention is adapted for forming a micro-emulsion (in the sparger), said micro-emulsion comprising pressurized (liquefied) disruption agent and pressurized suspension of biomass material. The (liquefied) disruption agent is the dispersed phase, and the suspension of biomass material is the continuous phase (or the dispersion medium) of the micro-emulsion.

More advantageously, the first openings (29,30,31,32) in said second sparger tube (22) are adapted (configured) for producing micro and/or nano (liquefied) disruption agent droplets (bubbles) during said injection, thereby producing (forming) a micro- and/or nano-emulsion of mixed (liquefied) disruption agent with biomass suspension inside the sparger. Accordingly, using the sparger of the present invention, there is a relatively high efficiency of mass transfer between the (liquefied) disruption agent and the suspension to be mixed in the sparger. In the micro- and/or nano-emulsion, micro and/or nano droplets (bubbles) of (liquefied) disruption agent (the dispersed phase) are dispersed in the suspension of biomass material (the continuous phase or dispersion medium).

More advantageously, the sparger system (7) according to the invention is adapted for forming a micro- and/or nano-emulsion (in the sparger), said micro- and/or nano-emulsion comprising pressurized (liquefied) disruption agent and pressurized suspension of biomass material. The (liquefied) disruption agent is the dispersed phase, and the suspension of biomass material is the continuous phase (or the dispersion medium) of the micro- and/or nano-emulsion.

During the injection using the sparger of the invention, micro and/or nano (liquefied) disruption agent droplets (bubbles) are passing through (or disruption agent is bubbling through) the pressurized suspension of biomass material flowing in the second sparger tube (22) (the disruption agent thereby getting in (physical) contact with the suspension of biomass material).

In the context of the present invention, micro ((liquefied) disruption agent) droplets (bubbles) are droplets (bubbles) smaller than one millimeter in diameter, but larger than one micrometer.

In the context of the present invention, nano ((liquefied) disruption agent) droplets (bubbles) are droplets (bubbles) smaller than one micrometer in diameter, but larger than one nanometer.

Advantageously, the second opening (27) in said second sparger tube (22) is adapted for further (or more effective) mixing micro and/or nano (liquefied) disruption agent droplets (bubbles) with the pressurized suspension of biomass material (thereby further (or more effective) mixing the micro- and/or nano-emulsion formed in the sparger).

Advantageously, the second opening (27) in said second sparger tube (22) serves as outlet (28) for the process stream (through which the process stream exits the sparger system (7) and enters the tubing system (8)).

In the context of the present invention, process stream refers to the mixed stream (formed in the sparger) of disruption agent and suspension of biomass.

It will be convenient to note that the sparger according to the invention fundamentally differs from those already described in prior art. Indeed, during injection of a first stream of pressurized (liquefied) disruption agent into a second stream of pressurized suspension of biomass material using (a) sparger system(s) already described in the art, only droplets of disruption agent having a diameter comparable to the tube diameter are produced (e.g. having a diameter of (about) 3 mm to (about) 6 mm). Accordingly, there is a (very) low efficiency of mass transfer between the disruption agent and the suspension to be mixed in the sparger. In other words, no micro- and/or nano-emulsion of disruption agent mixed with suspension is formed when using a sparger known in the art. In US 2011/0183403, for example, pressurized biogenic suspended raw material (pressurized suspension of biomass material) is first brought together with pressurized solvent (pressurized disruption agent) in one line to obtain a solution mixture under pressure. It is only then that the solution mixture is further injected (atomized) through injection jets directly into a cubicle (the cubicle having a lower pressure than the solution mixture, thereby performing pressure reduction and atomization of the solution mixture simultaneously). Using a sparger according to the present invention, to the contrary, relatively high efficiency of mass transfer is achieved (due to the formation of the micro and/or nano disruption agent droplets (bubbles)), forming a micro- and/or nano-emulsion of disruption agent mixed with suspension of biomass, in the sparger.

Advantageously, the sparger system (7) of the invention is further connected to the primary tube (15) of tubing system (8) through the second sparger tube (22) having (or via) a second opening (second orifice) (27).

Otherwise stated, the sparger system (7) is connected to the primary tube (15) of the tubing system (8) via the second opening (27) at the outlet (28) of the second sparger tube (22). Second opening (27) is serving as outlet of the second sparger tube (22). The primary tube (15) is further sequentially connecting the hydrodynamic agitation system (11) and the relief valve (13).

Preferably, diameter (d7) of primary tube (15) is comprised between (about) 0.4 mm (1/64 inch) and (about) 12.7 mm (1/2 inch), more preferably between (about) 0.8 mm (1/32 inch) and (about) 12.7 mm (1/2 inch), even more preferably between (about) 1.6 mm (1/16 inch) and (about) 3.2 mm (1/8 inch), most preferably diameter (d7) of primary tube (15) is (about) 3.2 mm (1/8 inch).

Advantageously, the tubing system (8) (cf. e.g. **figure 1a**) is adapted for further mixing the process stream coming out the sparger system (7), thereby forming a (one) homogeneously mixed process stream (i.e. a homogenized mixture of disruption agent and suspension of biomass material, or a homogenized micro- and/or nano-emulsion of disruption agent with biomass suspension).

The apparatus comprising a sparger system (7) of the present invention can be provided with or without including a pressure oscillation system (9).

More advantageously, the tubing system (8) further comprises a (or at least one) pressure oscillation system (9) before the (upstream from) (at least one) hydrodynamic agitation system (11). More particularly, the sparger system (7) is connected via primary tube (15) to a (or to at least one) pressure oscillation system (9) which is further connected (by said tube (15)) to the (at least one) hydrodynamic agitation system (11).

An example of an apparatus further comprising a pressure oscillation system (9) is schematically illustrated in **figure 1b****.**

Preferably, the pressure oscillation system (9) comprises at least two (i.e. two, three, or more) orifices (in, or inside, primary tube (15)) connected in series (by tube (15), primary tube (15) is thus comprised inside pressure oscillation system (9)). The orifices of the pressure oscillation system (9) are in fluid communication with_the primary tube (15).

More preferably, the pressure oscillation system (9) comprises (or consists of) three orifices connected in series (by tube (15), primary tube (15) is thus comprised inside pressure oscillation system (9)).

Preferably, each orifice in the pressure oscillation system (9) has a diameter (d8), said diameter (d8) being (about) four to (about) sixty-four times smaller (i.e. the ratio (d8)/(d7) ranges from (about) 1/64 to (about) 1/4), more preferably being (about) eight to (about) twenty-three times smaller (i.e. the ratio (d8)/(d7) ranges from (about) 1/32 to (about) 1/8), than the diameter (d7) of the primary tube (15).

Preferably, the diameter (d8) of the orifices in the pressure oscillation system (9) is comprised between (about) 0.02 and (about) 0.4 mm, more preferably between (about) 0.2 and (about) 0.4 mm.

More preferably, the diameter (d8) of one, or two, or three, or more orifices of the pressure oscillation system (9) can be equal or different with respect to each other. In other words, (one, or two, or three, or more) orifices of the pressure oscillation system (9) can have a same or a different diameter (d8).

Even more preferably, the diameter (d8) of the three orifices of the pressure oscillation system (9) can be equal or different with respect to each other. In other words, the three orifices of the pressure oscillation system (9) can have a same or a different diameter (d8).

Finding suitable diameters (d8) and (d9) of the orifice(s) and of the primary tube (15), respectively, in the pressure oscillation system (9), depends on the characteristics of the disruption agent and of the suspension of biomass material used.

In the context of the present invention, an orifice of the pressure oscillation system (9) refers to a means for provoking Venturi effect. More particularly, such orifice is designed for controlling the characteristics of the stream flowing through it (especially to increase velocity and to reduce the pressure, due to the Bernoulli's equation) as it exits the orifice (and subsequently enters the next orifice or the primary tube). An orifice has a varying cross sectional area (compared to the primary tube), and is used to modify the flow of a fluid, for controlling the rate of flow, speed, and pressure of the stream that emerges from them.

In the present description, pressure oscillation system (9) can also be referred to as pressure and velocity oscillation system (or pressure/velocity system or pressure/velocity zone).

Advantageously, the pressure oscillation system (9) is adapted for further mixing the micro- and/or nano-emulsion formed in the sparger, thereby further increasing the efficiency of mass transfer between the (liquefied) disruption agent and the suspension already mixed in the sparger by further transferring the energy in between pressure-velocity-pressure oscillations, and enhancing the efficiency of the (resulting) cell disruption (i.e. the cell disruption achieved when the micro- and/or nano-emulsion has further passed the hydrodynamic agitation system (11) and the relief valve (13)).

Using the pressure oscillation system (9) is decreasing the cell number (i.e. more cells are being disrupted) and thus changing the final release ratio of cellular substances (i.e. optimizing solubilization of cellular substances), when compared with only using a hydrodynamic agitation system (11) for mixing the process stream.

More particularly, the extraction ratios of the cellular substances initially comprised in the biomass material can be tuned using the apparatus comprising the sparger system (7) of the invention provided with or without a pressure oscillation system (9).

In the apparatus comprising the sparger system (7) of the present invention, a standard hydrodynamic agitation system (11) known in the art is used.

Advantageously, the hydrodynamic agitation system (11) comprises at least one (i.e. one, two, three, or more) subsidiary tubing system (16) connected by the (at least one) primary tube (15), wherein the diameter (d9) of the tubes of the subsidiary tubing system (16) is one to two times smaller (i.e. the ratio (d9)/(d7) ranges from 0.5 to 1), more advantageously two times smaller (i.e. the ratio (d9)/(d7) is 0.5), than the diameter (d7) of the primary tube (15) (in case the process flow splits in two).

More advantageously, the hydrodynamic agitation system (11) comprises (or consists of) three subsidiary tubing systems (16,17,18) connected by the (at least one) primary tube (15), wherein the diameter (d9) of the tubes of the subsidiary tubing systems (16,17,18) is one to two times smaller (i.e. the ratio (d9)/(d7) ranges from 0.5 to 1), even more advantageously two times smaller (i.e. the ratio (d9)/(d7) is 0.5), than the diameter (d7) of the primary tube (15) (in case the process flow splits in two).

More advantageously, the diameter (d9) of one, or two, or three, or more subsidiary tubing systems (16) of the hydrodynamic agitation system (11) can be equal or different with respect to each other. In other words, (one, or two, or three, or more) subsidiary tubing systems (16) of the hydrodynamic agitation system (11) can have a same or a different diameter (d9).

Even more advantageously, the diameter (d9) of the three tubing systems (16) of the hydrodynamic agitation system (11) can be equal or different with respect to each other. In other words, the three subsidiary tubing systems (16) of the hydrodynamic agitation system (11) can have a same or a different diameter (d9).

Preferably, the diameter (d9) of the tubes of the subsidiary tubing system(s) (16,17,18) is comprised between (about) 0.4 mm (1/64 inch) and (about) 12.7 mm (1/2 inch), more preferably between (about) 0.4 mm (1/64 inch) and (about) 6,4 mm (1/4 inch), even more preferably diameter (d9) of the tubes of the subsidiary tubing system(s) (16,17,18) is (about) 1.6 mm (1/16 inch) (in case the process flow splits in two).

Advantageously, the hydrodynamic agitation system (11) is adapted for further mixing (or hydrodynamic mixing) the process stream, thereby increasing the mass transfer of pressurized disruption agent to the suspension of biomass material and producing a more homogeneous micro- and/or nano-emulsion.

In the context of the present invention, a relief valve (13) refers to a means or valve to control or limit the pressure in a system or in a vessel.

In the apparatus comprising the sparger system (7) of the present invention, the relief valve (13) is adapted for (suddenly) decreasing the pressure in the system, i.e. to decrease the pressure of the process stream passing through and coming out of the relief valve (13) to reduced pressure or to atmospheric pressure.

Using the apparatus, and passing a micro- and/or nano-emulsion of (liquefied) disruption agent with biomass suspension formed in the sparger system (7) of the present invention sequentially through a hydrodynamic agitation system (11) and a relief valve (13), thereby exposing the biomass material to an explosive (or rapid) decompression (i.e. suddenly drop in pressure), results in disrupting the treated biomass material.

Optionally, using the apparatus, the micro- and/or nano-emulsion of disruption agent with biomass suspension formed in the sparger system (7) of the present invention is first passed through a pressure oscillation system (9) before sequentially passing through a hydrodynamic agitation system (11) and a relief valve (13). Thereby the biomass material is exposed to an explosive (or rapid) decompression, resulting in disruption of the treated biomass material.

Advantageously, in the apparatus, the tubing system (8) (after the sparger system (7) of the present invention) further comprises at least one means (12) for measuring pressure.

Preferably, said means (12) for measuring pressure is comprised (or present) in between the (at least one) hydrodynamic agitation system (11) and the relief valve (13) (and connected to them via primary tube (15)).

Advantageously, the at least one means (12) for measuring (and monitoring) pressure is a pressure gauge.

Advantageously, in the apparatus, the first and second high-pressure pumps (10,20) comprise a pressure gauge.

Advantageously, in the apparatus, the relief valve (13) comprises a means (14) for heating a process stream coming out of the tubing system (8).

Advantageously, said relief valve (13) is further connected to a third vessel (35) by a (outflow) tube (33).

Advantageously, said third vessel (35) is used for collecting the process stream coming out of the relief valve (13).

In the context of the present invention, a means for heating (14) refers to a heating system being adapted for heating or increasing the temperature of a (homogeneously mixed) process stream (or output stream) coming out of the tubing system (8) (said heating system being able to resist to very high pressures).

Advantageously, the means (14) for heating comprised in relief valve (13) is adapted for increasing the temperature of the micro- and/or nano-emulsion (process stream coming out of the tubing system (8)) to avoid a (possible) freezing effect of rapid decompression (or to avoid freezing effect of pressure decrease, to avoid ice formation, due to using relief valve (13)).

Alternatively, the means (14) for heating comprised in relief valve (13) is adapted for increasing the temperature of the micro- and/or nano-emulsion (process stream coming out of the tubing system (8)) to the subcritical or critical point (or even higher temperature) of the disruption agent. Increasing the temperature of the micro- and/or nano-emulsion to the subcritical or critical point of the disruption agent, while transferring the process stream coming out of the relief valve (13) to a third vessel (35) (via tube (33)) at reduced or atmospheric pressure solubilizes (valuable) cellular substances (or cellular extracts) (from the disrupted biomass material).

According to an aspect not according to the present invention (but useful for the understanding thereof), an apparatus is thus provided for disrupting suspended biomass material and (optionally) solubilizing the (valuable) cellular substances (from the disrupted biomass material).

Preferably, the diameter (d10) of outflow tube (33) is comprised between (about) 0.1 mm and (about) 12.7 mm, more preferably between (about) 0.1 mm and (about) 3.175 mm.

Advantageously, the material used for the apparatus is cold manufactured stainless steel (or 304 SS, 304L SS, 316 SS, 316L SS)

An apparatus schematically illustrated in **figure 1a and 1b** comprising a sparger system of the invention can be used for disrupting suspended biomass material.

Advantageously, an apparatus schematically illustrated in **figure 1a and 1b** can be used for disrupting suspended biomass material and (optionally) solubilizing the (obtained, valuable) cellular substances.

The (solubilized) cellular valuable substances (from the disrupted biomass material) can be further (partially) isolated (or (partially) separated) and selective extracted.

Advantageously, (solubilized) proteins can be further (partially) isolated (or (partially) separated) and selective extracted from disrupted biomass material.

According to an aspect not according to the invention but useful for the understanding thereof, there is provided a stack (or arrangement) of (parallel) systems (devices, apparatuses) for disrupting suspended biomass material using a sparger system of the present invention.

Advantageously, a stack (or arrangement) of (parallel) systems (devices, apparatuses) can be used for disrupting suspended biomass material and (optionally) solubilizing the (obtained, valuable) cellular substances.

Advantageously, said stack (or arrangement) of (parallel) systems is based on the apparatus discussed in detail above.

A stack (or arrangement) of (parallel) systems not according to the present invention comprises a (one) first vessel for holding (or providing) a (untreated) suspension of biomass material connected to a (one) first high-pressure pump, said first high-pressure pump being adapted for pressurizing the suspension of biomass material coming from (supplied by) said first vessel; a (one) second vessel for holding (or providing) a (pressurized) gas disruption agent and/or (pressurized) liquid disruption agent connected to a (one) second high-pressure pump, said second high-pressure pump being adapted for (further) pressurizing the gas and/or liquid disruption agent coming from (supplied by) said second vessel; wherein the first and second high-pressure pumps are connected to a series of (two, three, or more) (parallel) systems via (using) a distribution manifold, each of said parallel systems comprising at least one sparger system of the present invention, said sparger system being adapted for injecting a first stream of pressurized disruption agent into a second stream of a pressurized suspension of biomass material and for forming a micro- (and/or nano-)emulsion, wherein said sparger system is further connected to a tubing system for mixing said first and second streams, said tubing system comprising a (at least one) primary tube and (a) at least one hydrodynamic agitation system, and said tubing system being further connected to a relief valve.

Each of the (two, three, or more) (parallel) systems can be provided with or without including a pressure oscillation system.

More advantageously, the tubing system in each of the (two, three, or more) (parallel) systems further comprises a (or at least one) pressure oscillation system before the (upstream from) (at least one) hydrodynamic agitation system.

Advantageously, the relief valve of each of the (two, three, or more) (parallel) systems is connected to a (one) third vessel via (using) a distribution manifold.

Advantageously, said third vessel is used for collecting the process streams coming out of each of the relief valves (of each of the (two, three, or more) (parallel) systems).

The stack (or arrangement) of (parallel) systems can be used on industrial scale.

According to an aspect not according to the invention but useful for the understanding thereof, there is provided a method for disrupting suspended biomass material, using a sparger system of the present invention.

Such a method for disrupting suspended biomass material comprises the steps of:
- providing a suspension of biomass material in a first vessel (1), said suspension having a dry cell weight being comprised between 0.1% and 15% (w/w);
- pressurizing said suspension to a (first) pressure (p1) being comprised between 7500 kPa and 450000 kPa by using a first high-pressure pump (10);
- providing a pressurized gas disruption agent and/or a pressurized liquid disruption agent in a second vessel (2) (the gas disruption agent and/or liquid disruption agent in the second vessel (2) being at a pressure (p0) comprised between (about) 5000 kPa (50 bar) and (about) 6000 kPa (60 bar));
- pressurizing said gas and/or liquid disruption agent to a (second) pressure (p2) being comprised between 7500 kPa and 450000 kPa by using a second high-pressure pump (20), said (second) pressure (p2) being higher than said (first) pressure (p1);
- injecting a stream of said pressurized disruption agent into a stream of said pressurized suspension of biomass material (by using a sparger system (7) according to the present invention, thereby bringing said two streams together) and forming a micro-emulsion;
- passing said micro-emulsion sequentially through a hydrodynamic agitation system (11) and a relief valve (13).

A (untreated) suspension of biomass material is provided in a first vessel (1), said suspension having a dry cell weight being comprised between (about) 0.1% and (about) 15% (w/w). A stream of said suspension is then flowing through tube (3) to a first high-pressure pump (10). Said suspension is then pressurized to a (first) pressure (p1) being comprised between (about) 7500 kPa (75 bar) and (about) 450000 kPa (4500 bar), by using the first high-pressure pump (10). Furthermore, a pressurized gas and/or pressurized liquid disruption agent is provided in a second vessel (2). The gas disruption agent and/or liquid disruption agent in the second vessel (2) is present in said vessel (2) at a pressure (p0) being comprised between (about) 5000 kPa (50 bar) and (about) 6000 kPa (60 bar). A stream of said pressurized gas disruption agent and/or pressurized liquid disruption agent is then flowing through tube (4) to a second high-pressure pump (20). Said gas and/or liquid disruption agent (coming from second vessel (2)) is then (further) pressurized to a (second) pressure (p2) being comprised between (about) 7500 kPa (75 bar) and (about) 450000 kPa (4500 bar), by using the second high-pressure pump (20). Said (second) pressure (p2) is higher than said (first) pressure (p1). A stream of said pressurized suspension of biomass material coming out of the first high-pressure pump (10) is then flowing through tube (5). A stream of said pressurized (liquefied) disruption agent coming out of the second high-pressure pump (20) is flowing through tube (6). The stream of said pressurized (liquefied) disruption agent is then injected (or sparged) into the stream of said pressurized suspension of biomass material (by using a sparger system (7) according to the invention, thereby bringing said two streams together) and forming a micro-emulsion (in the sparger system (7)). Said (formed) micro-emulsion is then sequentially passed through a hydrodynamic agitation system (11) and a relief valve (13).

The gas and/or liquid disruption agent coming from (supplied by) said second vessel (2) is only present in liquid phase (or liquid state) after having passed second high-pressure pump (20), said stream of pressurized disruption agent flowing after (or coming out of) second high-pressure pump (20) is thus only present in liquid phase (or liquid state, i.e. being liquefied).

Advantageously, a micro- and/or nano-emulsion is formed in the sparger system (7). Said (formed) micro- and/or nano-emulsion is then sequentially passed through a hydrodynamic agitation system (11) and a relief valve (13).

Preferably, the dry cell weight of the (untreated) suspension of biomass is comprised between (about) 0.1% and (about) 6% (w/w), more preferably between (about) 0.5 % and (about) 4 % (w/w), even more preferably between (about) 1.5 % and (about) 2.5 % (w/w).

Preferably, said (first) pressure (p1) is comprised between (about) 7500 kPa and (about) 25000 kPa, more preferably between (about) 20000 kPa and (about) 25000 kPa.

Preferably, said (second) pressure (p2) is comprised between (about) 7500 kPa and (about) 25000 kPa, more preferably between (about) 20000 kPa and (about) 25000 kPa.

Said (second) pressure (p2) is higher than said (first) pressure (p1).

The disruption agent in second vessel (2) is present in gas phase (or gas state) and/or in liquid phase (or liquid state), depending on the temperature and/or the pressure in the system.

Astream of pressurized disruption agent coming out of the second high-pressure pump (20) (flowing through tube (6) to sparger system (7)) is only present in liquid phase (or liquid state) (i.e. the pressure applied by second high-pressure pump (20) is such that no gas disruption agent is present in the system any more after having passed second high-pressure pump (20)).

Preferably, the superficial velocity of the mixed stream (or process stream) of disruption agent and suspension of the biomass material inside the tube (15) is lower than (about) 0.51 m/s, more preferably lower than (about) 0.1 m/s, even more preferably lower than (about) 0.05 m/s, and most preferably lower than (about) 0.03 m/s.

It is well within the practice of those skilled in the art that sample flow rates can be changed by changing the pressure in the system.

With the method, a same yield of cell disruption can be achieved using higher pressures combined with lower flow rates, or using lower pressures combined with higher flow rates.

With the method, (valuable) cellular substances from suspended biomass material with a high throughput (and low contact time) are obtained. It is believed that the high throughput (and low contact time) is due to the superficial velocities of disruption agent and suspension inside the tubes (5,6) of the system, the diameter (d7) of primary tube (15), and the diameter (d9) of the tubes of the subsidiary tubing system(s) (16,17,18) (in the hydrodynamic agitation system (11)).

Biomass material can be used to produce high-value bioactive molecules.

Advantageously, biomass material comprises (or consists of) biogenic source material.

More advantageously, said biomass material comprises (or consists of) single cell (or unicellular) biomass material.

Advantageously, the biomass material is suspended in a water-based environment (i.e. being in an aqueous suspension).

Advantageously, the suspension of biomass material comprises (or consists of) microalgae, bacteria, yeast/fungi cell, plant cell, animal cell, insect cell, or cyanobacteria.

Advantageously, the size of the particulates of the biomass material (cells) (in the suspension) is larger than (about) 1 µm.

Preferably, the ratio of the disruption agent to the suspension of biomass material is comprised between (about) 0.001 and (about) 0.95 kg/kg, more preferably between (about) 0.005 and (about) 0.95 kg/kg, even more preferably between (about) 0.18 kg/kg and (about) 0.5 kg/kg, and most preferably between (about) 0.23 kg/kg and (about) 0.45 kg/kg.

In the context of the present invention, the ratio of disruption agent to suspension (of biomass material) refers to the ratio of disruption agent mass flow rate to (biomass material) suspension mass flow rate.

It is to be noted that the total amount of disruption agent used is reduced, when compared to prior art methods. Consequently the method consumes less energy, thereby reducing the total operational expenditure (or total cost), when compared to prior art methods.

Advantageously, the sparger system (7) according to the invention is adapted for forming a micro-emulsion (in the sparger), said micro-emulsion comprising pressurized disruption agent and pressurized suspension of biomass material.

More advantageously, the sparger system (7) according to the invention is adapted for forming a micro- and/or nano-emulsion (in the sparger), said micro- and/or nano-emulsion comprising pressurized disruption agent and pressurized suspension of biomass material.

Referring to **figure 2****,** the stream of the pressurized (liquefied) disruption agent flowing in tube (6) is injected (or sparged) into the stream of the pressurized suspension of biomass material flowing in tube (5) by using a sparger system (7) of the invention, thereby bringing said two streams together. Tube (5) is connected to inlet (34) of the sparger system (7). Tube (6) is connected to inlet (24) of the sparger system (7).

More particularly, the stream of the pressurized (liquefied) disruption agent flowing in tube (6) is injected via inlet (24) of the sparger system (7) of the invention into the stream of the pressurized suspension of biomass material flowing through inlet (34) in the second sparger tube (22). The injection of the disruption agent into the suspension is performed through openings (29,30,31,32) through the surface (wall) of the second sparger tube (22).

Advantageously, the stream of said pressurized disruption agent is injected into the stream of said pressurized suspension through openings (29,30,31,32) (through the surface (wall)) of ((second) sparger tube (22) having) a (third) diameter (d3) comprised between (about) 0.01 mm and (about) 1 mm.

Preferably, the (third) diameter (d3) is comprised between (about) 0.01 mm and (about) 0.2 mm, more preferably between (about) 0.025 mm and (about) 0.085 mm, even more preferably between (about) 0.05 mm and (about) 0.06 mm.

Advantageously, the sparger system (7) of the invention is a high-pressure sparger system (a sparger system for high pressure processes). The pressure inside sparger system (7) is (second) pressure (p2), set by second high-pressure pump (20) and further depends on the disruption agent flow rate in tube (6) and the number and diameter of the (first) sparger holes.

Advantageously, after the step of using the sparger system (7) of the invention (i.e. after the step of forming the micro-emulsion) and before the step of passing sequentially through the hydrodynamic agitation system (11) and the relief valve (13), said micro-emulsion is passing through a pressure oscillation system (9).

More advantageously, after the step of using the sparger system (7) of the invention (i.e. after the step of forming the micro- and/or nano-emulsion) and before the step of passing sequentially through the hydrodynamic agitation system (11) and the relief valve (13), said micro- and/or nano-emulsion is passing through a pressure oscillation system (9).

Advantageously, said micro- and/or nano-emulsion is then passing through the sparger system (7) of the invention to the primary tube (15) of a pressure oscillation system (9) which is further connected (by said tube (15)) to the (at least one) hydrodynamic agitation system (11). More particularly, said micro- and/or nano-emulsion is passing through a second opening (27) of its second sparger tube (22) having diameter (d4) to the primary tube (15) of a pressure oscillation system (9) which is further connected to a hydrodynamic agitation system (11).

Preferably, said second opening (27) has a diameter (d4) comprised between (about) 0.01 mm and (about) 0.6 mm, more preferably between (about) 0.1 mm and (about) 0.6 mm.

Advantageously, the pressure oscillation system (9) is adapted for further mixing the micro- and/or nano-emulsion formed in the sparger of the present invention (thereby further increasing the efficiency of mass transfer between the (liquefied) disruption agent and the suspension already mixed in the sparger by further transferring the energy in between pressure-velocity-pressure oscillations and enhancing the efficiency of the (resulting) cell disruption). In other words, in the pressure oscillation system (9), the biomass material (comprised in the micro- and/or nano-emulsion passing through it) is prepared for actually being disrupted when subsequentially passing through the hydrodynamic agitation system (11) and exiting from the relief valve (13). Using the pressure oscillation system (9) is decreasing the cell number (i.e. more cells are being disrupted) and thus changing the final release ratio of cellular substances (i.e. optimizing solubilization of cellular substances), when compared with only using a hydrodynamic agitation system (11) for mixing the process stream.

Preferably, in the pressure oscillation system (9) the micro- and/or nano-emulsion passes through at least two orifice(s) (being connected in series by tube (15)), each orifice having a diameter (d8) being (about) four to (about) sixty-four times smaller (i.e. the ratio (d8)/(d7) ranges from (about) 1/64 to (about) 1/4), more preferably being (about) eight to (about) twenty-three times smaller (i.e. the ratio (d8)/(d7) ranges from (about) 1/32 to (about) 1/8), than the diameter (d7) of its primary tube (15).

Advantageously, the hydrodynamic agitation system (11) is adapted for further mixing (or hydrodynamic mixing) the process stream (thereby increasing the mass transfer of pressurized disruption agent to the suspension of biomass material and producing a more homogeneous micro- and/or nano-emulsion).

Advantageously, the micro- and/or nano-emulsion formed in sparger system (7) of the invention is passed through a means (12) for measuring pressure.

Advantageously, the micro- and/or nano-emulsion passes said means (12) for measuring pressure in between the hydrodynamic agitation system (11) and the relief valve (13).

More particularly, the micro- and/or nano-emulsion formed in sparger system (7) of the invention is further passing through a means (12) for measuring pressure after coming out of the hydrodynamic agitation system (11) and before passing through the relief valve (13).

The relief valve (13) (suddenly) decreases the pressure in the system, i.e. decreases the pressure of the process stream passing through and coming out of the relief valve (13) to reduced pressure or to atmospheric pressure.

In the context of the present invention, reduced pressure refers to a pressure being higher than atmospheric pressure and being lower than the pressure measured by the means (12) for measuring pressure (or by pressure gauge (12)) in between the hydrodynamic agitation system (11) and relief valve (13).

Passing a micro- and/or nano-emulsion of disruption agent with biomass suspension formed in the sparger system (7) of the present invention sequentially through a hydrodynamic agitation system (11) and a relief valve (13), thereby exposing the biomass material to an explosive (or rapid) decompression (i.e. suddenly drop in pressure), results in disrupting the treated biomass material.

Advantageously, the relief valve (13) comprises a means (14) for heating a process stream coming out of the tubing system (8) and passing through the relief valve (13).

In other words, after having formed a micro- and/or nano-emulsion in the sparger system (7) of the invention, and sequentially passing said micro- and/or nano-emulsion through a hydrodynamic agitation system (11) and a relief valve (13), the biomass material (comprised in the micro- and/or nano-emulsion) is being disrupted.

Optionally, the micro- and/or nano-emulsion of disruption agent with biomass suspension formed in the sparger system (7) of the invention is first passed through a pressure oscillation system (9) before sequentially passing through a hydrodynamic agitation system (11) and a relief valve (13). Thereby the biomass material is exposed to an explosive (or rapid) decompression, resulting in disruption of the treated biomass material.

More particularly, in an aspect useful for understanding the invention a method is provided for disrupting suspended biomass material.

By passing a micro- and/or nano-emulsion (formed in sparger system (7) of the invention) sequentially through a hydrodynamic agitation system (11) and a relief valve (13), and optionally first passing through a pressure oscillation system (9) before sequentially passing through a hydrodynamic agitation system (11) and a relief valve (13), no high shear forces (or stress) act on the biomass material to be disrupted. Therefore, a non-mechanical, physical, mild (i.e. more gentle) disruption of biomass material is provided (when compared to cell disruption methods described in the art).

Preferably, the suspension of biomass material is saturated by the pressurized disruption agent by hydrodynamic mixing.

Alternatively, the suspension of biomass material is oversaturated by the pressurized disruption agent by hydrodynamic mixing.

Alternatively, the suspension of biomass material is not saturated by the pressurized disruption agent by hydrodynamic mixing.

In the context of the present invention, pretreatment may (further) reduce the energy requirement, and may avoid micronization of cell debris, and influence cell deactivation.

Preferably, the suspension of biomass material is pretreated by centrifugation, scrubbing, washing, filtering, crushing, grinding, ball mill, bead mill, high pressure homogenizer, high speed homogenizer, ultrasonic homogenizer, microwave, ultrasonic bath, french press, pulsed electric fields, pulsed arc, microfluidizer, or heat only treatment, prior to be provided in the first vessel (1).

More preferably, the suspension of biomass material is pretreated by scrubbing, bead mill, high pressure homogenizer, high speed homogenizer, or microwave, prior to be provided in the first vessel (1).

Alternatively, the suspension of biomass material is not pretreated.

Advantageously, the temperature of the micro- and/or nano-emulsion passing the relief valve (13) is increased (by the means (14) for heating comprised in said relief valve (13)) to avoid a (possible) freezing effect of rapid decompression (or to avoid freezing effect of pressure decrease, to avoid ice formation, due to using relief valve (13)).

Alternatively, simultaneously with (i.e. during, together with) the step of passing the micro- and/or nano-emulsion through the relief valve (13), the temperature of the micro- and/or nano-emulsion is increased to the subcritical or critical point (or even higher temperature) of the disruption agent and the process stream coming out of the relief valve (13) is transferred to a third vessel (35) (via tube (33)) at reduced or atmospheric pressure (using relief valve (13)) (thereby solubilizing (valuable) cellular substances (or cellular extracts)). Increasing the temperature of the micro- and/or nano-emulsion to the subcritical or critical point of the disruption agent is performed by a means (14) for heating comprised in said relief valve (13).

Advantageously, the reduced pressure is comprised between (about) atmospheric pressure and the pressure (in the system) in between the hydrodynamic agitation system (11) and relief valve (13).

In an aspect useful for understanding the invention a method is thus provided for disrupting suspended biomass material and (optionally) solubilizing the (valuable) cellular substances (from the disrupted biomass material).

More particularly, it is provided a method for disrupting suspended biomass material, by means of a combination of pressurization and hydrodynamic mixing with, subsequently, a liquid to supercritical to gas phase transition (or phase transition of pressurized liquid disruption agent to supercritical and subsequently to gas phase) for solubilizing (valuable) cellular substances.

More particularly, a method is provided for disrupting suspended biomass material and (optionally) subsequently solubilizing the (valuable) cellular substances (from the disrupted biomass material).

Advantageously, valuable cellular extracts (or cellular substances) from substance class of fat, oil, and lipids contained in the biomass material are solubilized.

Alternatively, valuable cellular extracts (or cellular substances) from substance class of carbohydrates contained in the biomass material are solubilized.

Alternatively, valuable cellular extracts (or cellular substances) from substance class of proteins contained in the biomass material are solubilized.

Alternatively, valuable cellular extracts (or cellular substances) from substance class of carotenoids contained in the biomass material are solubilized.

Obtained cellular substances can be used as nutrients, vitamins, for conversion by bacteria in digestions, or in food and pharmaceutical industry (e.g. antibiotics, enzymes, biochemicals).

Preferably, the steps of the method before the step of increasing the temperature to the subcritical or critical point of the disruption agent are performed at a temperature range between (about) 2°C up to (about) 70°C, more preferably between (about) 20°C up to (about) 70°C, even more preferably between (about) 20°C up to (about) 25°C, most preferably at room temperature.

More particularly, the steps of the method for disrupting the biomass material are performed at a temperature range between (about) 2°C up to (about) 70°C, more preferably between (about) 20°C up to (about) 70°C, even more preferably between (about) 20°C up to (about) 25°C, most preferably at room temperature.

By performing the method for disrupting biomass material, no extreme temperatures are applied. Therefore, (in addition to the absence of high shear forces acting on the biomass material to be disrupted) a mild (i.e. more gentle) disruption of biomass material is provided (when compared to cell disruption methods described in the art).

Advantageously, the disruption agent is recovered (out of or from the third vessel (35)) and returned into the second pump (20) (to be pressurized again). More particularly, the disruption agent is recycled (thereby (further) reducing the total amount of disruption agent used, and reducing the total operational expenditure (or total cost), compared to prior art methods).

Advantageously, the disruption agents used are known to be used in refrigerant systems and/or in supercritical fluid extractions. Finding a suitable disruption agent for use in the invention depends on the product to be obtained from the biomass material.

Advantageously, the disruption agent is selected from the group consisting of ethane, propane, butane, ethylene, propylene, butylene, other saturated or unsaturated hydrocarbons, carbon dioxide, nitrous oxide, dimethyl ether, sulfur hexafluoride, freon, and mixtures thereof.

More advantageously, freon is including chlorofluorocarbons, such as R141 b; or hydrofluorocarbons, such as R134a, R125, or R32.

Alternatively, the disruption agent is not being a hydrocarbon (consisting entirely of hydrogen and carbon atoms).

Alternatively, the disruption agent is a hydrocarbon further comprising one or several heteroatom(s), said heteroatom (preferably) being selected from the group consisting of oxygen, nitrogen, sulfur, and/or one or several halogen(s).

Advantageously, a further step of (partially) isolating (or (partially) separating) and selective extracting (the solubilized) cellular valuable substances (from disrupted biomass material) is performed.

More particularly, a further step of (partially) isolating (or (partially) separating) and selective extracting (solubilized) proteins (from disrupted biomass material) is performed.

More advantageously, after performing the mild disruption and subsequent solubilization, (the solubilized) proteins are further (partially) isolated (or (partially) separated) and selective extracted from the disrupted biomass material.

More advantageously, the (final) treated suspension (of biomass material, comprising solubilized cellular valuable substances) (passing the relief valve (13) or being present in the third vessel (35)) is (partially) separated by centrifugation and/or filtration, so as to obtain soluble (containing proteins and carbohydrates) and insoluble (being lipid enriched) phases.

In other words, after cell disruption and (optionally) solubilization, supernatant and solid phases are separated by centrifugation and/or filtration.

More advantageously, the (final) treated suspension (passing the relief valve (13) or being present in the third vessel (35)) is separated in a lipid enriched insoluble phase and a soluble phase containing proteins and carbohydrates by centrifugation and/or filtration.

Even more advantageously, the (final) treated suspension is separated (in a lipid enriched insoluble phase and a soluble phase containing proteins and carbohydrates) by centrifugation.

Advantageously, the method is integrated with other cell disruption methods for increasing the efficiency of the disruption of biomass (however, thereby still assuring an overall mild cell disruption).

Advantageously, the other cell disruption methods are performed on the micro- and/or nano-emulsion coming out of the tubing system (8), but before performing the step of increasing the temperature of the emulsion to the subcritical or critical point of the disruption agent (while transferring it to a third vessel (35) at reduced or atmospheric pressure). Suitable cell disruption methods for integration will be apparent for those skilled in the art.

Preferably, other cell disruption methods are selected from a group of mechanical methods consisting of cell disruption by means of ball mill, bead mill, high pressure homogenizer, high speed homogenizer, ultrasonic homogenizer, microwave, ultrasonic bath, french press, pulsed electric fields, pulsed arc, and microfluidizer. More preferably, other cell disruption methods are selected from a group of mechanical methods consisting of cell disruption by means of high pressure homogenizer, microwave, ultrasonic bath, pulsed electric fields, and microfluidizer.

Alternatively, other cell disruption methods are selected from a group of chemical methods consisting of cell disruption by means of antibiotics, chelating agents, chaotropes, detergents, hypochlorites, other co-solvents, acid and alkali treatment.

Alternatively, other cell disruption methods are selected from a group of biological methods consisting of cell disruption by means of enzymes, phages, and autolysis.

Alternatively, other cell disruption methods are selected from a group of physical methods consisting of cell disruption by means of freezing and thawing, thermolysis, and explosive decompression.

### EXAMPLES

In the examples described below useful for understanding the invention, the dimensions of the apparatus used (when including a pressure oscillation system and a sparger system) are the following:
distance from first pump to sparger = 45 cm;
distance from second pump to sparger = 45 cm;
length of pressure oscillation system = 10 cm (i.e. starting from 1^{st} orifice + 3 cm of primary tube + 2^{nd} orifice + 3 cm of primary tube + 3^{rd} orifice + 4 cm of primary tube);
length of hydrodynamic agitation system = 3 subsidiary tubing system of 10*10 cm² each with 2.5 cm primary tube in between them;
distance from hydrodynamic agitation zone to relief valve = 45 cm.

Other suitable dimensions of the apparatus used will be apparent for those skilled in the art.

### Example 1 - Not according to the invention:

### Solubilization ratio (%) between untreated and treated suspension(s) of biomass

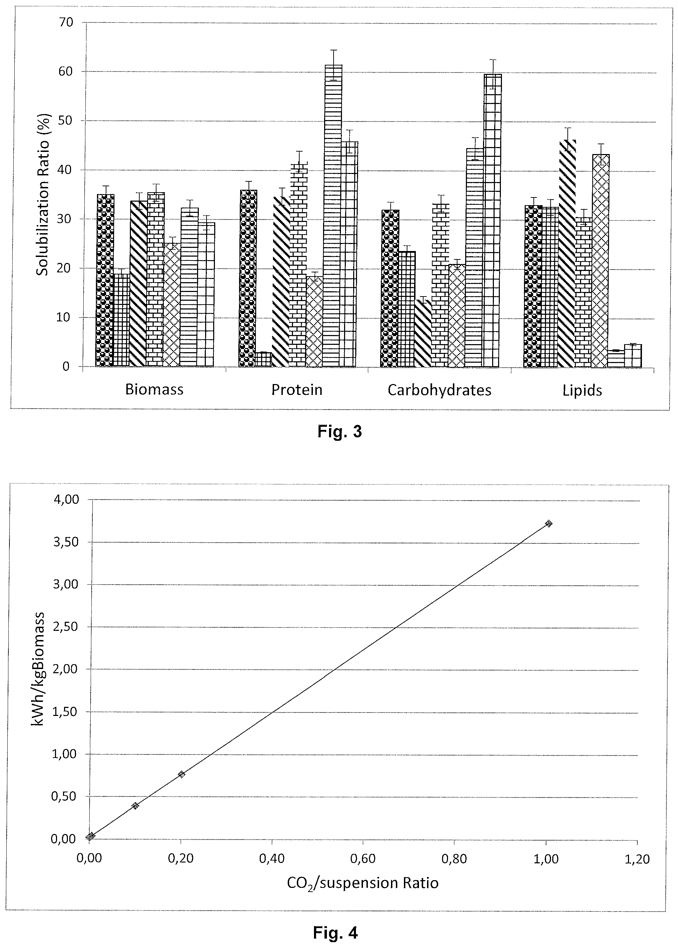

Suspensions of *Neochloris oleoabundans* (unicellular, green microalgae) biomass were treated either by conventional bead milling, conventional explosive decompression method, or the explosive (or rapid) decompression method not according to an aspect of the invention (performed with or without including a pressure oscillation system). The suspension was also treated by a preliminary explosive (or rapid) decompression method wherein both a sparger system and a pressure oscillation system were not included. After cell disruption and solubilization, the treated suspensions were analyzed to determine the total biochemical compositions, i.e. total lipid, protein, and carbohydrate determinations were carried out. Also the untreated sample of green microalgae biomass was analyzed. The results are summarized in **figure 3****.**

Three runs of **conventional bead milling** were performed on algae suspensions having a dry cell weight of 2.81 % (w/w), 3.59 % (w/w), and 5.84 % (w/w), respectively. The algae suspension was recirculated through the grinding chamber of the agitation bead mill DYNO®-Mill, Type MULTI LAB, (supplied by Willy A. Bachofen AG Maschinenfabrik, Muttenz, Switzerland) for 30 min with a pump speed of 1.5 L/min. The 0.3 L grinding chamber was filled with the maximum amount (approximately 65 % [v/v]) of ceramic beads made of Zirconia, Yttria stabilized, 0.4-0.6 mm. Water, cooled to 5 °C, was continuously circulating in the cooling jacket of the grinding chamber to avoid elevated temperatures inside the chamber. Outside the grinding chamber the algae suspension was kept at 5 °C. Using these process conditions the temperature of the algae suspension at the mill outlet never exceeded 20 °C. The conventional bead milling average results are depicted by 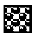 in **figure 3****.**

The **conventional explosive decompression method** is performed inside 100 ml premex high-pressure stirred vessel reactors, at 35°C stable temperature and 10000-20000 kPa (100-200 bar) pressure. Treatment time is 15-45 min, during which 100-300 rpm stirring is performed on a suspension having a (initial) dry cell weight of 6.4 % (w/w). 5 runs are performed to see the effect of the parameters. The best results from conventional explosive decompression runs are depicted by 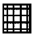 in **figure 3****.**

Performing the **explosive decompression method without including a pressure oscillation system and without including a sparger system** (also referred to as the **preliminary explosive decompression method, without pressure** **oscillation and without sparger),** the **highest biomass solubilization run** was performed with a CO₂/suspension ratio (w/w) of 241 %, while the relief valve temperature set to 70 °C, the suspension having a dry cell weight of 3.2 % (w/w), the cell suspension flow rate being 4,15 g/min at a pressure of 200 bar, and without any co-solvent addition. 33.7 % of biomass is solubilized. The biochemical composition of the solubilized material was the same (± 3) with untreated biomass. The result from the related run is depicted by in **figure 3****.**

It is to be noted that in the preliminary explosive decompression method, the total amount of disruption agent used increases a lot, when compared to the method performed including a sparger system of the present invention. In other words, the total amount of disruption agent used is reduced performing the method including a sparger system, when compared to the preliminary explosive decompression method without pressure oscillation and without sparger.

**Highest biomass and lipid solubilization was achieved in the method performed without pressure oscillation** (or in other words, the explosive decompression method without pressure oscillation). The **highest biomass solubilization run** was performed with a CO₂/suspension ratio (w/w) of 20 %, while the whole system was at room temperature, the suspension having a dry cell weight of 2.6 % (w/w), the cell suspension flow rate being 15 g/min at a pressure of 250 bar, and without any co-solvent addition. 35.41 % of biomass is solubilized. 45.73 % of the solubilized material was protein, while only 35.22 % of the untreated biomass was protein. The best results from the highest biomass solubilization run are depicted by 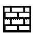 in **figure 3****. The highest lipid solubilization run** was performed with a CO₂/suspension ratio (w/w) of 19.46 %, the hydrodynamic agitation system being cooled down to 4°C, the relief valve being at room temperature, the suspension having a dry cell weight of 2.5 % (w/w), the cell suspension flow rate being 4.8 g/min at a pressure of 75 bars, and with 8 % (w/w) isopropanol addition as co-solvent. 43.43 % of lipids are solubilized. 58.2 % of the solubilized material was lipid. The best results from the highest lipid solubilization run are depicted by 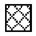 in **figure 3****.**

**Highest protein and carbohydrate solubilization was achieved in the method performed with pressure oscillation** (or in other words, the explosive decompression method with pressure oscillation). **The highest protein solubilization run** was performed with a CO₂/ suspension ratio (w/w) of 25.08 %, while the relief valve is at 45°C, the suspension having a dry cell weight of 1.65 % (w/w), the cell suspension flow rate being 10.5 g/min at a pressure of 175 bar, and without any co-solvent addition. 61.47 % of proteins are solubilized. 77.7 % of the solubilized material were proteins, while only 40.86 % of the untreated biomass were proteins. The best results from the highest protein solubilization run are depicted by 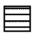 in **figure 3****.** The **highest carbohydrate solubilization run** was performed with a CO₂/ suspension ratio (w/w) of 50.71 %, while the relief valve is at 20°C, the suspension having a dry cell weight of 1.65 % (w/w), the cell suspension flow rate being 15 g/min at a pressure of 225 bar, and without any co-solvent addition. 59.65 % of carbohydrates are solubilized. 41.9 % of the solubilized material were carbohydrates, while only 20.6 % of the untreated biomass were carbohydrates. The best results from the highest carbohydrate solubilization run are depicted by 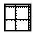 in **figure 3****.**

The data in **figure 3** show that the distribution of biochemicals can be tuned up by changing process parameter values and/or by modifying the design parameters. Using the conventional methods, the system favored lipids, however, the lipid extraction yield was not enough.

In the method useful for understanding the invention, cells are enriched at higher liquefied gas pressures (like liquid CO₂) instead of high (but still directly) gas pressure as in prior art conventional explosive decompression methods.

From this example it follows that the extraction ratios of the cellular substances initially comprised in the biomass material can be tuned (thereby minimizing the contaminating load).

### Example 2 - Not according to the invention:

### Energy consumption (in kWh/kg of dry biomass) versus CO₂/suspension of biomass material ratio

A suspension of *Neochloris oleoabundans* (unicellular, green microalgae) biomass having 2 % DCW (dry cell weight) concentration is treated using the cell disruption method not according to an aspect of the invention.

Bernoulli's equation is used to compute the energy of the fluids before entering the system and when being in the system. The energy, which should be added to the system, to provide a pressure of 250 bar to a 15 g/min single cell suspension and a 0.075-15 g/min CO₂ flow (at room temperature), is calculated by subtracting the total energy in the system and before the system. For the energy calculation, the starting conditions of both the single cell suspension and the CO₂ are assumed being at atmospheric pressure at room temperature.

**Figure 4** represents the energy consumption (in kWh/kg of dry biomass) *versus* the CO₂/suspension ratio, showing that the energy consumption performing the method is comprised between (about) 0.04 kWh/kgDryBiomass and (about) 3.54 kWh/kgDryBiomass while the ratio of the disruption agent to the suspension of single cell biomass material is ranging between (about) 0.005 to (about) 0.950 kg/kg.

In prior art, to the contrary, much higher amounts of solvent (disruption agent) compared to the amount of biomass (slurry) material are used.

Indeed, for example in US 2011/0183403, it is described that the ratio of solvent (disruption agent) to biogenic suspended raw material (18.1 % DCW) is ranging between 1 kg/kg and 90 kg/kg. The specific examples in said document only mentioning 90 kg/kg and 110 kg/kg (CO₂/suspended biogenic raw material) (with unknown amount at 1500 bar and unknown amount at 300 bar), meaning more energy is consumed when performing the prior art method for disrupting biomass material described in US 2011/0183403.

Using the sparger system of the invention in a method for disrupting suspended biomass material and related apparatus, the total amount of disruption agent used can thus be reduced and consequently less energy is consumed, thereby reducing the total operational expenditure (or total cost), compared to prior art methods.

From the description and the examples above, it follows that the present invention thus provides an improved sparger system which can be used in a method for disrupting suspended biomass material and a related apparatus, which overcome the disadvantages of prior art methods and apparatuses.

In particular, the present invention provides a sparger system which can be used in a method and a related apparatus for a physical, non-mechanical, mild disruption of suspended biomass material compared to prior art methods and apparatuses.

Furthermore, using the sparger system of the invention in a method and related apparatus for disrupting suspended biomass material, (valuable) cellular substances from suspended biomass material with a high throughput (and low contact time) can be obtained.

The disruption of suspended biomass material can (optionally) be integrated with a solubilization and/or subsequent selective extraction or separation of the obtained (valuable) cellular substances.

More particularly, a solubilization of cellular substances from suspended biomass material and minimization of contaminating load can be provided by tuning the extraction ratio of the preferred product or biochemical.

Moreover, the total operational expenditure (or total cost) can be lowered compared to prior art methods and apparatuses and being applicable on industrial scale.

Using the sparger system of the present invention thus provides a more efficient method compared to prior art methods.

It will be apparent that the stack of (parallel) systems (said stack being based on the apparatus comprising the sparger system of the present invention) overcomes the disadvantages of prior art apparatuses as well.

## Claims

1. A sparger system, comprising:
a first tube (21) comprising a first inlet (24) for supplying a stream of pressurized disruption agent into the tube (21);
a second tube (22) comprising a second inlet (34) for supplying a stream of a pressurized suspension of biomass material through the tube (22), the second tube (22) comprising an outlet (28),
wherein the second tube (22) traverses the first tube (21) and comprises at least one set (23) of first openings through a wall of the tube (22), the first openings being in fluid communication with the first tube (21) such that a micro-emulsion is formed by forcing the stream of pressurized disruption agent through the first openings into the second tube (22),
wherein the first openings have a third diameter (d3) between 0.01 mm and 1 mm.

2. The sparger system of claim 1, wherein the first tube (21) is dead ending on the second tube (22).

3. The sparger system of claim 1 or 2, wherein the first tube (21) has a first diameter (d1) comprised between 1.6 mm and 25.4 mm.

4. The sparger system of any of claim 1 to 3, wherein the second tube (22) has a second diameter (d2) comprised between 0.8 mm and 12.7 mm.

5. The sparger system of claim 1 or 4, wherein the ratio of the second diameter (d2) of the second tube (22) to the first diameter (d1) of the first tube (21) is 0.5.

6. The sparger system of any of claim 1 to 5, wherein the second tube (22) comprises a second opening (27) at the outlet (28) of the second tube (22), said second opening (27) having a diameter (d4) comprised between 0.01 mm and 0.6 mm.

7. The sparger system of any of claim 1 to 6, wherein each set (23) of first openings in second tube (22) comprises at least two first openings (29,31), said openings being located opposite each other in one plane, said plane being perpendicular to the second tube (22), the first openings (29,31) being located through the wall of the second tube (22) at a distance D1 satisfying the equation D1=(diameter (d2) of second tube x π)/2 from the next opening of the set.

8. The sparger system of any of claim 1 to 7, wherein each set (23) of first openings in second tube (22) comprises at least four first openings (29-32), said openings being located two by two opposite each other in one plane, said plane being perpendicular to the second tube (22), each of the first openings (29-32) being located through the wall of the second tube (22) at a distance D1 satisfying the equation D1=(diameter (d2) of second tube x π)/4 from the next opening of the set.

## Patentansprüche

1. Sprengungssystem (10), umfassend:
ein erstes Rohr (21) umfassend einen ersten Einlass (24) zum Zuführen eines Stroms von druckbeaufschlagtem Spaltmittel in das Rohr (21);
ein zweites Rohr (22) umfassend einen zweiten Einlass (34) zum Zuführen eines Stroms einer druckbeaufschlagten Suspension von Biomassematerial durch das Rohr (22), wobei das zweite Rohr (22) einen Auslass (28) umfasst,
wobei das zweite Rohr (22) das erste Rohr (21) durchquert und mindestens einen Satz von ersten Öffnungen durch eine Wand des Rohres (22) umfasst, wobei die ersten Öffnungen in Fluidverbindung mit dem ersten Rohr (21) sind, derart dass eine Mikroemulsion durch Drücken des Stroms von druckbeaufschlagtem Spaltmittel durch die ersten Öffnungen in das zweite Rohr (22) gebildet wird,
wobei die ersten Öffnungen einen dritten Durchmesser (d3) zwischen 0,01 mm und 1 mm aufweisen.

2. Das Sprengungssystem nach Anspruch 1, wobei das erste Rohr (21) am zweiten Rohr (22) blind endet.

3. Das Sprengungssystem nach Anspruch 1 oder 2, wobei das erste Rohr (21) einen ersten Durchmesser (d1) im Bereich von 1,6 mm bis 25,4 mm aufweist.

4. Das Sprengungssystem nach irgendeinem der Ansprüche 1 bis 3, wobei das zweite Rohr (22) einen zweiten Durchmesser (d2) im Bereich von 0,8 mm bis 12,7 mm aufweist.

5. Das Sprengungssystem nach Anspruch 1 oder 4, wobei das Verhältnis des zweiten Durchmessers (d2) des zweiten Rohres (22) zum ersten Durchmesser (d1) des ersten Rohres (21) 0,5 beträgt.

6. Das Sprengungssystem nach irgendeinem der Ansprüche 1 bis 5, wobei das zweite Rohr (22) eine zweite Öffnung (27) am Auslass (28) des zweiten Rohres (22) umfasst, wobei die besagte zweite Öffnung (27) einen Durchmesser (d4) im Bereich von 0,01 mm bis 0,6 mm aufweist.

7. Das Sprengungssystem nach irgendeinem der Ansprüche 1 bis 6, wobei jeder Satz (23) von ersten Öffnungen im zweiten Rohr (22) mindestens zwei erste Öffnungen (29, 31) umfasst, die besagte Öffnungen einander in einer Ebene gegenüberliegend angeordnet sind, die besagte Ebene senkrecht auf das zweite Rohr (22) ist, die ersten Öffnungen (29, 31) durch die Wand des zweiten Rohres (22) in einem Abstand D1, der die Gleichung D1 = (Durchmesser (d2) des zweiten Rohres x π)/2 erfüllt, von der nächsten Öffnung des Satzes angeordnet sind.

8. Das Sprengungssystem nach irgendeinem der Ansprüche 1 bis 7, wobei jeder Satz (23) von ersten Öffnungen im zweiten Rohr (22) mindestens vier erste Öffnungen (29-32) umfasst, die besagte Öffnungen einander in einer Ebene paarweise gegenüberliegend angeordnet sind, die besagte Ebene senkrecht auf das zweite Rohr (22) ist, jede der ersten Öffnungen (29-32) durch die Wand des zweiten Rohres (22) in einem Abstand D1, der die Gleichung D1 = (Durchmesser (d2) des zweiten Rohres x π)/4 erfüllt, von der nächsten Öffnung des Satzes angeordnet ist.

## Revendications

1. Système asperseur, comprenant :
un premier tube (21) comprenant une première entrée (24) pour fournir un flux d'agent de disruption sous pression dans le tube (21) ;
un second tube (22) comprenant une seconde entrée (34) pour fournir un flux d'une suspension de matière de biomasse sous pression à travers le tube (22), le second tube (22) comprenant une sortie (28),
dans lequel le second tube (22) traverse le premier tube (21) et comprend au moins un ensemble (23) de premières ouvertures à travers une paroi du tube (22), les premières ouvertures étant en communication fluidique avec le premier tube (21) de telle sorte qu'une micro-émulsion est formée en forçant le flux d'agent de disruption sous pression à travers les premières ouvertures dans le second tube (22),
dans lequel les premières ouvertures ont un troisième diamètre (d3) entre 0,01 mm et 1 mm.

2. Système asperseur selon la revendication 1, dans lequel le premier tube (21) vient mourir sur le second tube (22).

3. Système asperseur selon la revendication 1 ou 2, dans lequel le premier tube (21) a un premier diamètre (d1) compris entre 1,6 mm et 25,4 mm.

4. Système asperseur selon l'une quelconque des revendications 1 à 3, dans lequel le second tube (22) a un deuxième diamètre (d2) compris entre 0,8 mm et 12,7 mm.

5. Système asperseur selon la revendication 1 ou 4, dans lequel le rapport du deuxième diamètre (d2) du second tube (22) sur le premier diamètre (d1) du premier tube (21) est de 0,5.

6. Système asperseur selon l'une quelconque des revendications 1 à 5, dans lequel le second tube (22) comprend une seconde ouverture (27) à la sortie (28) du second tube (22), ladite seconde ouverture (27) ayant un diamètre (d4) compris entre 0,01 mm et 0,6 mm.

7. Système asperseur selon l'une quelconque des revendications 1 à 6, dans lequel chaque ensemble (23) de premières ouvertures dans le second tube (22) comprend au moins deux premières ouvertures (29, 31), lesdites ouvertures étant situées à l'opposé l'une de l'autre dans un seul plan, ledit plan étant perpendiculaire au second tube (22), les premières ouvertures (29, 31) étant situées à travers la paroi du second tube (22) à une distance D1 satisfaisant l'équation D1 = (diamètre (d2) du second tube x π)/2 de l'ouverture suivante de l'ensemble.

8. Système asperseur selon l'une quelconque des revendications 1 à 7, dans lequel chaque ensemble (23) de premières ouvertures dans le second tube (22) comprend au moins quatre premières ouvertures (29-32), lesdites ouvertures étant situées deux par deux à l'opposé les unes des autres dans un seul plan, ledit plan étant perpendiculaire au second tube (22), chacune des premières ouvertures (29-32) étant située à travers la paroi du second tube (22) à une distance D1 satisfaisant l'équation D1 = (diamètre (d2) du second tube x π)/4 de l'ouverture suivante de l'ensemble.
